(19)

**Europäisches Patentamt**

**European Patent Office**

**Office européen des brevets**

(11) **EP 4 180 559 A1**

(12) **EUROPEAN PATENT APPLICATION**

(43) Date of publication:
**17.05.2023 Bulletin 2023/20**

(21) Application number: **21383015.1**

(22) Date of filing: **10.11.2021**

(51) International Patent Classification (IPC):
*D04H 1/728* (2012.01)　　　*D01D 5/00* (2006.01)
*A61K 9/00* (2006.01)　　　*D01F 1/10* (2006.01)

(52) Cooperative Patent Classification (CPC):
**D04H 1/728; A61K 9/7007; A61K 31/00;
D01D 5/0007; D01F 1/10**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**
Designated Extension States:
**BA ME**
Designated Validation States:
**KH MA MD TN**

(71) Applicants:
• **Asociacion Centro Tecnologico Ceit**
　**20018 Donostia - San Sebastian (Guipuzcoa) (ES)**
• **Servicio Andaluz de Salud**
　**41071 Sevilla (ES)**
• **Universidad de Sevilla**
　**41013 Sevilla (ES)**

(72) Inventors:
• **MITXELENA IRIBARREN, OIHANE**
　**20018 DONOSTIA - SAN SEBASTIAN (GUIPUZCOA) (ES)**

• **MUJIKA GARMENDIA, MAITE**
　**20018 DONOSTIA - SAN SEBASTIAN (GUIPUZCOA) (ES)**
• **RIERA PONS, MARC**
　**20018 DONOSTIA - SAN SEBASTIAN (GUIPUZCOA) (ES)**
• **ARANA ALONSO, SERGIO**
　**20018 DONOSTIA - SAN SEBASTIAN (GUIPUZCOA) (ES)**
• **PADILLO RUIZ, JAVIER**
　**41013 SEVILLA (ES)**
• **CASTILLO TUÑÓN, JUAN MANUEL**
　**41013 SEVILLA (ES)**
• **CALERO CASTRO, FRANCISCO JOSE**
　**41013 SEVILLA (ES)**
• **PEREIRA ARENAS, SHEILA**
　**41013 SEVILLA (ES)**

(74) Representative: **Cueto, Sénida**
　**SP3 Patents S.L.**
　**Los Madroños, 23**
　**28891 Velilla de San Antonio (ES)**

(54) **DRUG DELIVERY SYSTEM USING ELECTROSPINNING OF POLYMERS**

(57)　The present invention refers to a nonwoven fibre mat formed by biocompatible nanofibers, characterized in that the biocompatible nanofibers comprises:
an outer layer composed by at least one biocompatible polymer, and
an inner layer, composed by molecules of at least one antineoplastic agent and at least one biocompatible polymer, the inner layer being completely surrounded by the outer layer, so that the antineoplastic agent is encapsulated within the nanofibers,
being both layers arranged concentrically being the diameter length of the outer layer between 35% and 65% of the total nanofiber diameter length, and the diameter length of the inner layer between 35% and 65% of the total nanofiber diameter length.

EP 4 180 559 A1

FIG. 9

**Description**

**FIELD OF THE INVENTION**

The invention relates to the field of drug delivery systems (DDS). In particular, the invention relates to a nonwoven fibre mat for the controlled and sustained release of an active agent in the area of the body to be treated. This nonwoven fibre mat comprises biocompatible electrospun nanofibers and antineoplastic molecules which are entangled between the nanofibers.

**BACKGROUND**

Cancer is one of the deadliest diseases worldwide. One of the worst prognoses of this disease is for the Pancreatic Cancer, as only the 4% of the patients survive after 5 years of the diagnosis date. It is also the second deadliest cancer-related cause in Western countries. Surgery, chemotherapy such as FOLFIRINOX or Gemcitabine, and radiotherapy are the most common treatments used to treat this illness. However, they have strong disadvantages such as side effects, limitations of techniques and recurrence due to rests of cancer non removed during the treatment, among others.

These disadvantages and limitations can be reduced if Drug Delivery Systems (DDS) are used during the healing process. The main advantage of these technologies is a focalized and more efficient delivery, which derives on a reduction in the side effects and tumour recurrence. Polymers are the main vehicles used for DDS because of the wide range of macromolecules that can be used. Each of them has a specific biodegradation profile, which can provide a controlled delivery as the degradation kinetics will describe the drug release profile. For example, Polycaprolactone (PCL) is a biodegradable polymer widely used as DDS, even with encapsulated anticancer drugs.

DDS can be divided into two groups, depending on the liberation profile they have: they can do it systematically (such as the case of nanoparticles) or locally (as in the case of electrospun fibres, films or hydrogels). Systemic delivery systems can be injected or introduced orally. The main advantages of these systems are their nanometric size, specificity and low invasiveness, as the vehicles can reach smaller vessels, can be engineered to recognize certain receptors and no surgery is required for the treatment. On the other hand, sometimes these particles are hard to functionalize or can lose their specificity due to the lack of molecules that can identify the target and the interactions in the delivery process.

Local drug delivery systems release the drug in a specific site, loco-regionally, by diffusion or degradation mainly. These systems can take several forms, such as foams, hydrogels or fibres among others. The main disadvantage of hydrogels or foams is that the drug is delivered by diffusion, which is a fast occurring phenomenon that releases the drug in a short period of time. Even more, the target of local DDS is not accessible without surgery. That is why these devices are meant to be complementary to conventional surgery, in order to ensure that the entire tumour is removed.

Electrospinning is a fabrication process to obtain a local DDS. This method is easy to use and versatile, it generates high surface to volume ratio meshes and it usually is a low-cost process. The generated scaffolds are formed by fibres, which have a mixed delivery profile, as both diffusion and degradation related release occur. The shape and size of the fibres play an important role in the drug releasing profile. Therefore, there are several parameters that can be altered to modify the size of those electrospun fibres in order to control the drug release, such as potential difference, nozzle-collector distance, polymeric solution flow, polymer concentration and conductivity. One limitation of this technique is that the fibre has the same properties across its whole structure and a burst drug release can occur, as the molecule is totally blended with the fibre. This can be solved if two solutions are injected at the same time by coaxial electrospinning, increasing the versatility and the control on the coaxial fibre properties, composition and drug loading and release.

Actual chemotherapy treatments use various drugs, such as 5-Fluorouracil (5F), irinotecan, oxaliplatin and folinic acid at the same time to treat pancreatic cancer cells. In addition, including other drugs such as painkillers, in the treatment could improve the patient's life quality. Therefore, testing PCL to encapsulate drugs related to general cancer treatment, such as Methotrexate (MTX), is a required step to keep DDS on the same road that chemotherapy is undergoing.

For this reason, in order to obtain an alternative treatment for pancreatic cancer, the present invention discloses a nonwoven fibre mat of PCL to encapsulate and release different antineoplastic molecules by coaxial electrospinning.

The patent document EP2644191 discloses a nonwoven fibre mat that comprises a biocompatible polymer and at least one active agent. However, the nonwoven fibre mat of the present invention allows for a controlled and sustained release of the antineoplastic compounds.

The scientific article *Polycaprolactone/chitosan blend nanofibers loaded by 5-fluorouracil: An approach to anticancer drug delivery system* discloses a nonwoven fibre *mat comprising PCL/chitosan and 5-F* discloses, a nonwoven fibre mat comprising PCL/chitosan and 5-F. However, the present invention discloses coaxial electrospinning unlike this document which discloses simple electrospinning.

The scientific article *Encapsulation of anticancer drugs (5-Fluorouracil and Paclitaxel) into polycaprolactone*

*(PCL) nanofibers and in vitro testing for sustained and targeted therapy* discloses a nonwoven fibre mat comprising PCL and 5-F. However, the antineoplastic agent of the present invention is solved in the polymeric solution and not in formic acid. This differences increase fibre stability as well as allow a tighter regulation of the compound release speed and sustained in time.

## DESCRIPTION OF THE INVENTION

In the context of the invention the expression "nonwoven fibre mat" relates to one or more polymeric electrospun nanofibers deposited arbitrarily onto a solid surface (collector) and optionally entangled by one or more of the following methods: physical methods such as adhesion with biocompatible glue, melting or solvent excess, and mechanic methods such as needle punch, hydroentanglement and neumatic entanglement.

In the context of the invention the expression "integrated" or "encapsulated" are synonyms and relates to the fact that molecules are entrapped, that is, physically retained in such a way that they cannot be released but in their solubilized form.

In the context of the invention the terms "scaffolds", "meshes" and "mats" are synonyms.

The present invention refers in the first place to a nonwoven fibre mat formed by biocompatible nanofibers, characterized in that the biocompatible nanofibers comprises:

an outer layer composed by at least one biocompatible polymer, and

an inner layer, composed by molecules of at least one antineoplastic agent and at least one biocompatible polymer, the inner layer being completely surrounded by the outer layer, so that the antineoplastic agent is encapsulated within the nanofibers,

being both layers arranged concentrically, being the diameter length of the outer layer between 35% and 65% of the total nanofiber diameter length, and the diameter length of the inner layer between 35% and 65% of the total nanofiber diameter length, as a result, the release of the antineoplastic agent is done in a controlled and sustained manner.

In the context of the invention the expression "controlled and sustained manner" means that:

7 days after the fibre mat is made or located within an internal body area, at least 70 % in weight of the antineoplastic agent is still within the nonwoven fiber mat biocompatible nanofibers. Or, it means that a maximum of 30 % in weight of the antineoplastic agent has been released within the first 7 days, and/or

14 days after the fibre mat is made or located within an internal body area, at least 63 % in weight of the antineoplastic agent is still within the nonwoven fiber mat biocompatible nanofibers. Or, it means that a maximum of 37% in weight of the antineoplastic agent has been released within the first 14 days.

The outer layer diameter length is measured from the side facing the inner layer to the side facing the outside. Thus to determine the diameter length of a biocompatible nanofibre, the outer layer diameter length must be multiplied by 2 and added to the inner layer diameter length.

In a particular embodiment, the diameter length of the outer layer is equal or higher than 42% of the total nanofiber diameter length, and the diameter length of the inner layer is equal or lower than 58%, preferably, the diameter length of the outer layer is equal or higher than 45% of the total nanofiber diameter length, and the diameter length of the inner layer is equal or lower than 55%, more preferably the diameter length of the outer layer is equal or higher than 48% of the total nanofiber diameter length, and the diameter length of the inner layer is equal or lower than 52%, even more preferably, the diameter length of the outer layer is equal or higher than 50% of the total nanofiber diameter length, and the diameter length of the inner layer is equal or lower than 50%.

An expert in the field would know how to measure the diameter length of both the outer layer and the inner later. The measurement can be made with a Transmission Electron Microscopy preferably. Any of the following documents describe a protocol that can be applied to the biocompatible nanofibers of the present invention in order to assess the diameter length, although an expert can use any other technique and/or protocol well known and commonly used in the field: Coaxial Electrospinning and Characterization of Core-Shell Structured Cellulose Nanocrystal Reinforced PMMA/PAN Composite Fibers. Chao Li, Qingde Li, Xiaohui Ni, Guoxiang Liu, Wanli Cheng, and Guangping Han* Materials (Basel). 2017 Jun; 10(6): 572. Preparation and characterization of coaxial electrospun thermoplastic polyurethane/collagen compound nanofibers for tissue engineering applications Rui Chen, Chen Huang, Qinfei Ke, Chuanglong He, Hongsheng Wang, Xiumei Mo Colloids and Surfaces B: Biointerfaces, Volume 79, Issue 2, 2010, Pages 315-325, ISSN 0927-7765. Preparation and Characterization of Coaxial Electrospinning rhBMP2-Loaded Nanofiber Membranes He Zhao, Yali Ma, Dahui Sun, Wendi Ma, Jihang Yao, Mei Zhang Journal of Nanomaterials, vol. 2019, Article ID 8106985, 13 pages, 2019 and Coaxial electro-spun PEG/PA6 composite fibers: Fabrication and characterization Aziz Babapoor, Gholamreza Karimi, Seyyed Iman Golestaneh, Mehdi Ahmadi Mezjin. Applied Thermal Engineering, Volume 118, 2017, Pages 398-407, ISSN 1359-4311.

In a particular embodiment the biocompatible nanofibers have at least one of the following parameter values: diameter length between 70 and 400 microns, preferably between 85 and 350 microns, more preferably between 100 and 300 microns, the melting point is between 45°C and 80°C, preferably between 57°C and 70°C and the porosity is between 50% to 75%

In another particular embodiment the outer layer biocompatible polymer is selected from poly-glycolic acid, poly-D,L-lactic acid, poly-D,L-lactide-co-glycolide acid, polycaprolactone (PCL), polydioxanone, polyethylene glicol (PEG), polyurethanes (PU), polybenzimidazole (PBI), polycarbonate (PC), polyacrylonitrile (PAN), polyvinyl alcohol (PVA), polyethylene oxide (PEO), polystirene (PS), polyethylene terephtalate (PET), polyamide (PA), polyviniylphenol (PVP), polyvinylchloride (PVA) and any combination thereof.

In another particular embodiment the inner layer biocompatible polymer is selected from poly-glycolic acid, poly-D,L-lactic acid, poly-D,L-lactide-co-glicolide acid, polycaprolactone, polydioxanone and any combination thereof.

In another particular embodiment the outer layer and the inner layer comprise one or more common biocompatible polymers.

In an additional particular embodiment, the inner layer comprises polyvinyl acetate.

The antineoplastic agent of the inner layer may be at least one anticancer drug selected from:

- DNA-damaging agents,
- mitosis inhibitors that do not directly affect DNA,
- external agents that affect cell cycle,
- monoclonal antibodies, and/or
- other anticancer agents.

DNA (Deoxyribonucleic acid) damaging agents are defined herein as any chemical compound that induces DNA damage when applied to a cell. Therefore, they affect the integrity of nucleic acid chains, and therefore prevent normal replication.

In a particular embodiment the antineoplastic agent is at least one DNA damaging agent selected from the groups:

- alkylating agents such as nitrogenated mustards: bendamustine, cyclophosphamide, ifosfamide and/or melphalan; nitrosureas: carmustine, estramustine, and/or fotemustine; aziridines such as thiotepa; alkylsulphonates such as busulfan; triazenes such as dacarbazine and/or temozolomide; methylhydrazines such as procarbazine; and/ or
- platinum derivatives such as cisplatin, carboplatin, and/or oxaliplatin; and/ or
- antimetabolites such as pyrimidine analogues: azacitidine, capecitabine, cytarabine, decitabine, 5 fluorouracil, gemcitabine, mercaptopurine, tegafur, and/or trifluridine-tipiracil; purine analogues such as: cladribine, clofarabine, fludarabine, nelarabine and/or pentostatin; folic acid analogues such as: methotrexate, pemetrexed and/or raltitrexed and/or
- DNA interclating agents such as Anthracyclines and analogues: daunorubicin, doxorubicin, epirubicin, idarubicin, mitoxantrone and/or pixantrone; and/or
- cytotoxic antibiotics such as bleomycin and/or mitomycin; and/or
- camptothecins such as irinotecan and/or topotecan; and/or
- ascidins such as trabectedin,
- podophyllotoxin derivatives such as etoposide, and/or
- any combination thereof.

In another particular embodiment the antineoplastic agent is at least one mitosis inhibitor that do not directly affect DNA selected from the groups:

- Vinca alkaloyds agents such as Vinblastine, Vincristine, Vindesine, Vinorelbine, and/or Vinflunine;
- Taxanes such as Cabazitaxel, Docetaxel, and/or Paclitaxel;
- Halichondrins such as Eribulin, and/or
- any combination thereof.

In another particular embodiment the antineoplastic agent is at least one external agents that affect cell cycle selected from the groups:

- antiestrogens such as Flurvestrant, Tamoxifen, Anastrozole, Exemestane, and/or Letrozole;
- antiandrogens such as Flutamide, Bicalutamide, Enzalutamide, and/or Abiraterone;
- GnRH analogs such as Buserelin, Goserelin, Histrelin, Leuprorelin, and/or Triptorelin;
- GnRH antagonists such as Degarelix;
- Progestogens such as Medroxyprogesterone, and/or Megestrol
- Kinases inhibitors such as Afatinib, Axitinib, Baricitinib, Bosutinib, Cabozantinib, Cobimetinib, Crizotinib, Dabrafenib, Dasatinib, Erlotinib, Gefitinib, Ibrutinib, Idelasilib, Imatinib, Lapatinib, Lenvatinib, Nilotinib, Nintedanib, Olaparib, Pazopanib, Ponatinib Regorafenib, Ruxolitinib, Sorafenib, Sunitinib, Tofacitinib Trametinib Vandetanib, and/or Vemurafenib
- Rapamycin derivatives such as Everolimus, and/or Temsirolimús;
- Necrosis Tumor Factors such as Tasonermin;
- Interleukins such as Aldesleukin, and/or
- any combination thereof.

In another particular embodiment the antineoplastic agent is at least one monoclonal antibody or recombinant fusion proteins selected from: Aflibercept, Bevacizumab, Bretuximab-vedotina, Cetuximab, Ipilimumab, Nivolumab, Ofatumumab, Obinutuzumab, Panitumumab, Pembrolizumab, Pertuzumab, Ramucirumab, Rituximab, Siltuximab, Trastuzumab, and/or Trastuzumab emtansine.

In another particular embodiment the antineoplastic agent is at least one of the anticancer agents selected from: bacille Calmette-Guerin (BCG) vaccine, Bexarotene, Bortezomib, hydroxycarbamanide, Pegaspargase, arsenic trioxide, Vismodegib, 5-Aminolevulinic acid, methyl aminolevulinate, and/or Verteporfin.

An expert in the field would know which antineoplastic agent is more appropriate for an individual and a particular solid tumor using common general knowledge.

In a particular embodiment the DNA-damaging agent is selected from: camptothecin, topotecan, irinotecan (CPT-11), silatecan (DB-67, AR-67), cositecan (BNP-1350), exatecan, lurtotecan, gimatecan (ST1481), belotecan (CKD-602), rubitecan, and any combination thereof.

In a particular embodiment the antineoplastic agent is selected from at least one antimetabolite, and/or one camptothecin.

In another particular embodiment the antineoplastic agent is selected from 5-fluorouracil, methotrexate, irinotecan and any combination thereof.

In a particular preferred embodiment, the biocompatible polymer of the outer layer and inner layer is polycaprolactone and the antineoplastic agent of the inner layer is selected from 5-fluorouracil, methotrexate and irinotecan and any combination thereof.

In a particular embodiment the nonwoven fiber mat of the invention may additional comprise an adjuvant to prevent or minimize the antineoplastic drugs side effects and/or to increase its effectiveness such as leucovorin and/or one or more antibiotics selected from: Penicillin G, Penicillin V, Cloxacillin, Amoxicillin, Ampicillin, Ampicillin/sulbactam, Amoxicillin, Amoxicillin/clavulanic acid, Piperacillin, Piperacillin, Piperacillin/tazobactam, Cefadroxil, Cephalexin, Cephazolin, Cephachlor, Cephonicide, Cefoxitin, Cefuroxime, Cefditoren, Ceixime, Cefminox, Cefpodoxime, Ceftazidime, Ceftibutene, Ceftriaxone, Cefepime, Aztreonam, Imipenem, Meropenem, Ertapenem, Azithromycin, Clarithromycin, Erythromycin, Spiramycin, Josamycin, Midecamycin, Roxithromycin, Telithromycin, Lincomycin, Clindamycin, Chlortetracycline, Doxixicline, Minocycline, Oxytetracycline, Tetracycline, Tigecycline, Amikacin, Spectinomycin, Streptomycin, Gentamicin, Kanamycin, Neomycin, Tobramycin, Chloramphenicol, Bacitracin, Gramicidin, Vancomycin, Teicoplanin, Daptomycin, Polymyxin B, Colistin, Linezolid, Nitrofurantoin, Nitrofurazone, Metronidazole, Tinidazole, Fusidic acid, Fosfomycin, Retapamulin, Pipemidic acid Ciproloxacin, Levoloxacin, Moxiloxacin, Norloxacin, Oloxacin, Sulfadiazine, Sulfamethasazole, Sulfamethoxazole, Sulfanilamide, Pyrimethamine, Trimethoprim, Mupirocin and any combination thereof.

The adjuvant may be located in the inner layer

The present invention refers in the second place to a method of obtaining the nonwoven fibre mat comprising biocompatible electrospun nanofibers and molecules of at least one antineoplastic agent encapsulated inside the nanofibers described above comprising the steps of:

- coaxial electrospinning a:

(a) solution of at least one biocompatible polymer to form the outer layer; and

(b) a solution of molecules of at least one antineoplastic agent in a solution of at least one biocompatible polymer simultaneously, and

- desiccate the nonwoven fibre mat.

As known by the skilled person, electrospinning is a highly versatile technique which combines the use of two techniques namely electrospray and spinning. In the electrospinning process, a polymer solution held by its surface tension at the end of a capillary tube is subjected to an electric field and an electric charge is induced on the liquid surface due to this electric field. When the electric field applied reaches a critical value, the repulsive electrical forces overcome the surface tension forces. Eventually, a charged jet of the solution is ejected from the tip of the Taylor cone and an unstable and a rapid whipping of the jet occurs in the space between the capillary tip and collector which leads to evaporation of the solvent, leaving a polymer behind and leading to a mat of nanofibers deposited on a collector. The electrospinning process is controlled by a number of parameters, usually classified as: solution parameters, process parameters and environment parameters. Solution parameters include viscosity, conductivity, molecular mass and surface tension. Process parameters include the electric field applied, distance between capillary tube and collector and solution flow rate. Each one of these parameters has a significant influence on the morphology of nanofibers obtained by electrospinning process, and through proper manipulations of these parameters it could be possible to produce nanofibers with desirable morphology and diameters. Furthermore, the environment parameters include relative humidity and room temperature, which also have an important role on the morphology and diameters of electrospun nanofibers.

In another particular embodiment, the Molecular weight (Mw) of the biocompatible polymer can be from 10,000 g/mol to 500,000 g/mol, preferably from 20,000 g/ml to 200,000 g/mol, more preferably from 50,000 g/mol to 150,000 g/mol, even more preferably from 75,000 g/mol to 125,000 g/mol.

In a particular embodiment, the amount of polymer in the biocompatible polymer solution for electrospinning of step (a) can be from 5 to 50 weight%, preferably, 8 to 40 weight%, more preferably from 9 to 20 weight%, even more preferably from 9.5 to 15 weight % compared to the polymer solution.

In another particular embodiment, the amount of polymer in the biocompatible polymer solution for electrospinning of step (a) is 5 weight%, or 6 weight%, or 7 weight%, or 8 weight%, or 9 weight%, or 10 weight%, or 11 weight%, or 12 weight%, or 13 weight%, or 14 weight%, or 15 weight%, or 16 weight%, or 17 weight%, or 18 weight%, or 19 weight%, or 20 weight% compared to the polymer solution.

When the electrospinning solution of step (a) comprises more than one biocompatible polymer, the total amount can be from 5 to 50 weight%, preferably, 8 to 40 weight%, more preferably from 9 to 20 weight%, even more preferably from 9.5 to 15 weight % compared to the polymer solution, being the polymers in any proportion among them.

In another particular embodiment, the polymer in the biocompatible polymer solution for electrospinning of step (a) is polycaprolactone in an amount of 5 weight%, or 6 weight%, or 7 weight%, or 8 weight%, or 9 weight%, or 10 weight%, or 11 weight%, or 12 weight%, or 13 weight%, or 14 weight%, or 15 weight%, or 16 weight%, or 17 weight%, or 18 weight%, or 19 weight%, or 20 weight% compared to the polymer solution.

In an additional particular embodiment, the biocompatible polymer solution is polycaprolactone from 9.5 to 15 weight % compared to the polymer solution.

The polymer solution for electrospinning is prepared by dissolving the biocompatible polymer in a solvent selected from: DMSO, DMF (N,N-dimethylformamide), hydrochloric acid (HCl), TFE (2,2,2-Trifluoroethanol), HFIP (1,1,1,3,3,3-Hexafluoro-2-propanol), formic acid/acetic acid/chloroform and any combination thereof.

In a particular embodiment the solvent is formic acid/acetic acid/chloroform.

In another particular embodiment, the solvents formic acid/acetic acid/chloroform can have a ratio of 40-60:40-60:1-10 (v/v/v), respectively, preferably 45-55:45-55:2.5-7.5 (v/v/v), respectively.

In a particular preferred embodiment, the biocompatible polymer is polycaprolactone and the antineoplastic agent is selected from 5-fluorouracil, methotrexate, irinotecan and any combination thereof.

In a particular embodiment the resulting solution comprising PCL and the solvent acid/acetic acid/chloroform is stirred for a minimum time of 6 hours and used within the first 72 hours after it is made.

The solution of step (b) comprises at least one antineoplastic molecules which acts on DNA selected from the groups:

In a particular embodiment, the antineoplastic agent is 5-fluorouracil (5F) in a concentration between 10 and 50 mg/ml of the active agent, preferably between 15 and 40 mg/ml, more preferably between 20 and 35 mg/ml.

In another particular embodiment, the antineoplastic agent is methotrexate (MTX) in a concentration between 2 and 20 mg/ml of the active agent, preferably between 5 and 15 mg/ml, more preferably between 8 and 12 mg/ml.

In another particular embodiment, the antineoplastic agent is irinotecan in a concentration between 2 and 20 mg/ml of the active agent, preferably between 5 and 15 mg/ml, more preferably between 8 and 12 mg/ml.

In a particular embodiment, the nonwoven fibre mat of the invention comprises two antineoplastic agents.

In a particular embodiment, the nonwoven fibre mat of the invention comprises two active agents selected from: 5-fluorouracil, irinotecan and methotrexate.

In addition, as the chloroform is toxic, the nonwoven fibre mat obtained is desiccated at room temperature in a desiccator for a period ranging between 10hr and 72hr, preferably 12hr and 48hr, more preferably 24 hours and/or under vacuum. Therefore, the biocompatible polymer employed is 100 weight% of the nonwoven fibre mat excluding the amount of antineoplastic agent(s) and/or adyuvant(s) within the nonwoven fibre mat.

Once prepared the solution of biocompatible polymer, this is submitted to an electrospinning process in an electrospinning device that can be anyone of the art. For this purpose, the polymer solution is held by its surface tension at the end of a capillary tube and is subjected to an electric field. When the electric field applied reaches a critical value, the repulsive electrical forces overcome the surface tension forces. Eventually, a charged jet of the solution is ejected from the tip of the Taylor cone and an unstable and a rapid whipping of the jet occurs in the space between the capillary tip and collector which leads to evaporation of the solvent, leaving a polymer behind and leading to a mat of nanofibers deposited on the collector.

The high voltage tension or power supply applied to the jet was a critical value ranging from 20,5 kV to 27 kV, preferably from 20,5 kV to 25 kV, the best applied voltage found to improve the process was 21 kV. At this voltage electrospinning process is carried out uniformly, with the Taylor's cone formation, without bead defects and production of regular polymeric nanofibers with diameters in nanoscale. Additional optimal configuration was found for spinning flow rate, distance from spinneret to collector, size, shape and nature of collector, room temperature and humidity and spinning chamber geometry.

The process of the invention uses two different electrospinning flow rates for the outer layer and the inner layer.

In a particular embodiment the flow rates may be the same for both the outer and the inner layer.

The outer layer flow rate is ranging from 0,03 mL/h and 0,30 mL/h, preferably 0,05 mL/h and 0,25 mL/h, more preferably from 0,10 mL/h and 0,2 mL/h.

The inner layer flow rate is ranging from 0,03 mL/h and 0,30 mL/h, preferably 0,05 mL/h and 0,25 mL/h, more preferably from 0,05 mL/h and 0,2 mL/h, even more preferably it is 0,05 mL/h.

In a particular embodiment, the flow rate varies during the electrospinning procedure.

The electrospinning is carried out for a time between 0.5 h and 4 h, preferably between 0.75 h and 3.5 h, more preferably between 1 h and 3 h, even more preferably between 1.1 h and 2.75 h, even more preferably between 1.2 h and 2.5 h, even more preferably between 1.3 h and 2.25 hr.

The process of the invention uses two different electrospinning needles for the outer and the inner layer. The outer layer needle ranges from 15 G to 20 G, preferably between 16 G and 19 G. The inner layer needle ranges from 22 G to 30 G, preferably between 24 G and 28 G, more preferably between 25 G and 27 G.

The distance of the needle to the collector ranges from 5 cm to 25 cm, preferably from 10 cm to 20 cm, more preferably 11 cm to 17 cm, even more preferably between 12 cm to 15 cm. The collector is the base where the nanofibers are deposited.

The diameter of the nanofibers made by the conditions described above is less than 400 microns. In a preferred embodiment of the method of the invention, the diameter of the biocompatible nanofibers is in the range of 70 to 400 microns, preferably from 85 to 350 microns, more preferably 100 microns to 300 microns. In a particular embodiment, at least 60% of the biocompatible nanofibers are in the range of 85 to 350 microns, preferably at least 75 %, more preferably, at least 85% of the biocompatible nanofibers.

In another particular embodiment at least 90% of the biocompatible nanofibers are in the range of 100 microns to 300 microns.

The shape and dimensions of the nonwoven fibre mat obtained will be assessed by the expert according to the final purpose of it. Transmission Electron Microscopy and Scanning electron microscopy can be used using the common general knowledge and known protocols by an expert in the field.

In a particular embodiment, the conditions of the method of the invention are 21 Kv, voltage, a 0.15 mL/h outer flow rate, 0.05 mL/h inner flow rate, an 18 G outer layer needle, a 26 G inner layer needle and a 13.5 cm distance of the needle to the collector.

In another particular embodiment, the conditions of the method of the invention are 21 Kv, voltage, a 0.15 mL/h outer flow rate, 0.05 mL/h inner flow rate, an 18 G outer layer needle, a 26 G inner layer needle and a 14 cm distance of the needle to the collector.

In another particular embodiment, the conditions of the method of the invention are 21 Kv, voltage, a 0.15 mL/h outer flow rate, 0.1 mL/h inner flow rate, a 20 G outer layer needle, a 26 G inner layer needle and a 13.5 cm distance of the needle to the collector.

In another particular embodiment, the conditions of the method of the invention are 21 Kv, voltage, a 0.10 mL/h outer flow rate, 0.05 mL/h inner flow rate, an 18 G outer layer needle, a 27 G inner layer needle and a 14 cm distance of the needle to the collector.

In another particular embodiment, the conditions of the method of the invention are 21 Kv, voltage, between a 0,05 mL/h and 0,25 mL/h outer flow rate, 0.05 mL/h inner flow rate, an 18 G outer layer needle, a 26 G inner layer needle and a 13.5 cm distance of the needle to the collector.

In another particular embodiment, the conditions of the method of the invention are 21 Kv, voltage, between a 0,05 mL/h and 0,25 mL/h outer flow rate, 0.05 mL/h inner flow rate, an 18 G outer layer needle, a 26 G inner layer needle and a 12 cm to 15 cm distance of the needle to the collector.

In another particular embodiment, the conditions of the method of the invention are 21 Kv, voltage, a 0.15 mL/h outer flow rate, 0.05 mL/h inner flow rate, an 18 G outer layer needle, a 26 G inner layer needle and a 13.5 cm distance, the biopolymer is PCL and the antineoplastic agent is at least one anticancer drug selected from:

- DNA-damaging agents,
- mitosis inhibitors that do not directly affect DNA,
- external agents that affect cell cycle,
- monoclonal antibodies, and/or
- other agents.

In another particular embodiment, the conditions of the method of the invention are 21 Kv, voltage, a 0.15 mL/h outer flow rate, 0.05 mL/h inner flow rate, an 18 G outer layer needle, a 26 G inner layer needle and a 13.5 cm distance, the biopolymer is PCL and the antineoplastic agent is at least one DNA damaging drug selected from the groups:

- Alkylating Agents such as nitrogenated mustards: Bendamustine, cyclophosphamide, ifosfamide and/or melphalan; Nitrosureas: carmustine, estramustine, and/or fotemustine; Aziridines such as thiotepa; Alkylsulphonates such as busulfan; Triazenes such as dacarbazine and/or Temozolomide; Methylhydrazines such as procarbazine; and/ or
- Platinum derivatives such as cisplatin, carboplatin, and/or oxaliplatin; and/ or
- Antimetabolites such as pyrimidine analogues: azacitidine, capecitabine, cytarabine, decitabine, 5 fluorouracil, gemcitabine, mercaptopurine, tegafur, and/or trifluridine-tipiracil; Purine analogues such as: cladribine, clofarabine, fludarabine, nelarabine and/or pentostatin; Folic acid analogues such as: methotrexate, pemetrexed and/or raltitrexed and/or
- DNA interclating agents such as Anthracyclines and analogues: daunorubicin, doxorubicin, epirubicin, idarubicin, mitoxantrone and/or pixantrone; and/or
- Cytotoxic antibiotics such as bleomycin and/or mitomycin; and/or
- Camptothecins such as Irinotecan and/or Topotecan; and/or
- Ascidins such as Trabectedin, and/or
- Podophyllotoxin derivatives such as etoposide.

In another particular embodiment, the conditions of the method of the invention are 21 Kv, voltage, a 0.15 mL/h outer flow rate, 0.05 mL/h inner flow rate, an 18 G outer layer needle, a 26 G inner layer needle and a 13.5 cm distance of the needle to the collector, the biopolymer is PCL and the antineoplastic agent 5-FU.

In another particular embodiment, the conditions of the method of the invention are 21 Kv, voltage, a 0.15 mL/h outer flow rate, 0.05 mL/h inner flow rate, an 18 G outer layer needle, a 26 G inner layer needle and a 13.5 cm distance of the needle to the collector, the biopolymer is PCL and the antineoplastic agent MTX.

In another particular embodiment, the conditions of the method of the invention are 21 Kv, voltage, a 0.15-25 mL/h outer flow rate, 0.05 mL/h inner flow rate, an 18 G outer layer needle, a 26 G inner layer needle and a 13.5 cm distance of the needle to the collector, the biopolymer is PCL and the antineoplastic agent irinotecan.

In any of the previous three embodiments the biocompatible polymer solution is PCL from 9.5 to 15 weight %.

In addition, the melting point of the nonwoven fibre mat can be between 45°C and 80ºC, preferably between 57ºC and 70ºC.

An expert in the field would know how to adjust the remaining parameters or some of the to reach the object of the invention.

In another particular embodiment, when the nonwoven fiber mat comprises MTX, a decrease in the outer flow (ml/h) is directly proportional with a decrease in the biocompatible nanofibers diameter. Thus when the outer flow is 0,1 ml/h the diameter of the nonwoven fibre mat with MTX is 150 $\pm$ 50 microns.

In other aspect, the invention also relates to a nonwoven fibre mat obtainable by the method described above.

In a particular embodiment, the conditions of the method of the invention are: from 20 to 22 Kv, voltage, a 0.10 to 0.25 mL/h outer flow rate, 0.03 to 0.08 mL/h inner flow rate, an 18 G outer layer needle, a 26 G inner layer needle, and a 13 to 14 cm distance of the needle to the collector during 1.4 to 2.5 hours,

In other aspect the invention also relates to a nonwoven fibre mat as described above or obtainable by the method described above for use in medicine.

In other aspect, the invention also relates to the use of the nonwoven fibre mat previously defined or obtainable by the method defined above for the local delivery of an active ingredient in a controlled and sustained manner to an internal body area to be treated.

In the present specification "controlled and sustained manner" relates that at 7 days after the fibre mat is made or located within an internal body area, at least 72 % in weight of the antineoplastic agent is still within the fiber mat biocompatible nanofibers. Or, a maximum of 28% in weight of the antineoplastic agent has been released within the first 7 days, and/or

at 14 days after the fibre mat is made or located within an internal body area, at least 63 % in weight of the antineoplastic agent is still within the fiber mat biocompatible nanofibers. Or, a maximum of 37% in weight of the antineoplastic agent has been released within the first 14 days.

The internal body area to be treated must have previously been subjected to surgery to resect a solid tumor.

Local delivery of drugs loaded in the nonwoven membrane of the invention renders appropiate drug concentrations within the tumor bulk, reduces systemic drug exposure and is safe to surrounding healthy tissues. In addition, the product of invention allows for a spaced release of the antineoplastic agent, so the patient can recover from the surgery before the drug is started to be released by the degradation of the nonwoven membrane.

In a particular embodiment, the internal body area is selected from: mucosa, bones, muscles, internal organs, solid tumors and any combination thereof.

In another particular embodiment, the solid tumor is a solid tumor that needs to be resected from the internal body area for example: gastric adenocarcinoma, duodenal adenocarcinoma, mucinous cystoadenocarcinoma of the appendix, adenocarcinoma of the appendix, adenocarcinoma of the colon, adenocarcinoma of the rectum, epidermoid carcinoma of the rectum, adenocarcinoma of the anus, epidermoid carcinoma of the anus, colon adenocarcinoma, pancreatic cancer, hepatocarcinoma, hepatoblastoma, cholangiocarcinoma, hepatic cystoadenocarcinoma, hepatic epitheloid haemangioendothelioma, hepatic angiosarcoma, hepatic fibrosarcoma, hepatic leiomyosarcoma, hepatic metastases of colorectal carcinoma, hepatic metastases of endocrine carcinoma, hepatic metastases of non-colorectal and non-endocrine origin, pancreatic adenocarcinoma, serous cystoadenocarcinoma of pancreas, mucinous cystoadenocarcinoma of pancreas, papillary intraductal mucinous carcinoma of pancreas, acinar cell carcinoma of pancreas, pancreatoblastoma, pseudopapillary solid carcinoma, peritoneal carcinomatosis of ovarian origin, peritoneal carcinomatosis of gastric origin, peritoneal carcinomatosis of colorectal origin, pseudomyxoma peritoneum, mesothelioma, liposarcoma, malignant fibrohistiocytoma, leiomyosarcoma, paraganglioma, undifferentiated sarcoma, fibrosarcoma, retriperitoneal sarcoma, rhabdomyosarcoma, non-metastatic undifferentiated carcinoma, neuroblastoma, malignant schwanoma, benign schwanoma, ganglioneuroma, and/or lymphangioma.

In a preferred particular embodiment, the solid tumor is pancreatic cancer, preferably pancreatic adenocarcinoma.

The nonwoven fibre mat is useful for treating tumors that include non-resectable areas with vital vessels, for treating surgical borders wherein tumor residues are left, or for treating osseous tissues with tumor infiltration or positive bone scan.

The nonwoven fibre mat of the invention can be applied by overlapping the surface to be treated and then suturing the membrane borders to the surrounding tissue.

In addition, the present invention also provides the following clauses:

1. A nonwoven fibre mat formed by biocompatible nanofibers, characterized in that the biocompatible nanofibers comprise:

   an outer layer composed by at least one biocompatible polymer, and

   an inner layer, composed by molecules of at least one antineoplastic agent and at least one biocompatible polymer, the inner layer being completely surrounded by the outer layer, so that the antineoplastic agent is encapsulated within the nanofibers,

   being both layers arranged concentrically being the diameter length of the outer layer between 35% and 65% of the total nanofiber diameter length, and the diameter length of the inner layer between 35% and 65% of the total nanofiber diameter length.

2. The nonwoven fibre mat, according to the proceeding clause, wherein the diameter length of the outer layer is equal or higher than 42% of the total nanofiber diameter length, and the diameter length of the inner layer is equal or lower than 58% of the total nanofiber diameter length.

3. The nonwoven fibre mat according to any of the preceding clauses, wherein the biocompatible nanofibers have at least one of the following parameter value:

   a diameter length between 70 and 400 microns,

the melting point is between 45°C and 80ºC and/or

the porosity is between 50% to 75%

4. The nonwoven fibre mat according to any of the preceding clauses, wherein the outer layer biocompatible polymer is selected from poly-glycolic acid, poly-D,L-lactic acid, poly-D,L-lactide-co-glicolide acid, polycaprolactone (PCL), polydioxanone, polyethylene glycol (PEG), polyurethanes (PU), polybenzimidazole (PBI), polycarbonate (PC), polyacrylonitrile (PAN), polyvinyl alcohol (PVA), polyethylene oxide (PEO), polystirene (PS), polyethylene terephtalate (PET), polyamide (PA), polyviniylphenol (PVP), polyvinylchloride (PVA, polyvinyl acetate and any combinations thereof.

5. The nonwoven fibre mat according to any of the preceding clauses, wherein the inner layer biocompatible polymer is selected from poly-glycolic acid, poly-D,L-lactic acid, poly-D,L-lactide-co-glicolide acid, polycaprolactone (PCL), polydioxanone polyethylene glicol (PEG), polyurethanes (PU), polybenzimidazole (PBI), polycarbonate (PC), polyacrylonitrile (PAN), polyvinil alcohol (PVA), polyethylene oxide (PEO), polystirene (PS), polyethylene terephtalate (PET), polyamide (PA), polyviniylphenol (PVP), polyvinylchloride (PVA, polyvinyl acetate and any combinations thereof.

6. The nonwoven fibre mat according to any of the clauses 1 to 5, wherein the outer layer and the inner layer comprise one or more common biocompatible polymers.

7. The nonwoven fibre mat according to any of the clauses 1 to 6, wherein the inner layer comprises polyvinyl acetate.

8. The nonwoven fibre mat according to any of the clauses 1 to 7, wherein the antineoplastic agent is at least one anticancer drug selected from:

- DNA-damaging agents,
- mitosis inhibitors that do not directly affect DNA,
- external agents that affect cell cycle,
- monoclonal antibodies, and/or
- other anticancer agents.

9. The nonwoven fibre mat according to clause 8, wherein the antineoplastic agent is at least one DNA damaging agent selected from the groups:

- alkylating agents,
- platinum derivatives,
- antimetabolites,
- DNA interclating agents,
- cytotoxic antibiotics;
- camptothecins,
- ascidins,
- podophyllotoxin derivatives, and
  any combination thereof.

10. The nonwoven fibre mat according to any of clauses 8 to 9, wherein the DNA-damaging agent is selected from: camptothecin, topotecan, irinotecan (CPT-11), silatecan (DB-67, AR-67), cositecan (BNP-1350), exatecan, lurtotecan, gimatecan (ST1481), belotecan (CKD-602), rubitecan, and any combination thereof.

11. The nonwoven fibre mat according to any of clauses 8 to 10, wherein the DNA-damaging agent is selected from at least one antimetabolite, and/or one camptothecin.

12. The nonwoven fibre mat according to any of clauses 8 to 11, wherein the DNA-damaging agent is a camptothecin.

13. The nonwoven fibre mat according to any of the clauses 8 to 12, wherein the antineoplastic agent of the inner layer is selected from 5-fluorouracil, methotrexate, irinotecan and any combination thereof.

14. The nonwoven fibre mat according to any of the clauses 1 to 13, wherein the biocompatible polymer of the outer layer is polycaprolactone and the antineoplastic agent of the inner layer is selected from 5-fluorouracil, methotrexate and irinotecan and any combination thereof.

15. The nonwoven fibre mat according to any of the clauses 1 to 14, that comprises an adjuvant and/or one or more antibiotics selected from: Penicillin G, Penicillin V, Cloxacillin, Amoxicillin, Ampicillin, Ampicillin/sulbactam, Amoxicillin, Amoxicillin/clavulanic acid, Piperacillin, Piperacillin, Piperacillin/tazobactam, Cefadroxil, Cephalexin, Cephazolin, Cephachlor, Cephonicide, Cefoxitin, Cefuroxime, Cefditoren, Ceixime, Cefminox, Cefpodoxime, Ceftazidime, Ceftibutene, Ceftriaxone, Cefepime, Aztreonam, Imipenem, Meropenem, Ertapenem, Azithromycin, Clarithromycin, Erythromycin, Spiramycin, Josamycin, Midecamycin, Roxithromycin, Telithromycin, Lincomycin, Clindamycin, Chlortetracycline, Doxixicline, Minocycline, Oxytetracycline, Tetracycline, Tigecycline, Amikacin, Spectinomycin, Streptomycin, Gentamicin, Kanamycin, Neomycin, Tobramycin, Chloramphenicol, Bacitracin, Gramicidin, Vancomycin, Teicoplanin, Daptomycin, Polymyxin B, Colistin, Linezolid, Nitrofurantoin, Nitrofurazone, Metronidazole, Tinidazole, Fusidic acid, Fosfomycin, Retapamulin, Pipemidic acid Ciproloxacin, Levoloxacin, Moxiloxacin, Norloxacin, Oloxacin, Sulfadiazine, Sulfamethasazole, Sulfamethoxazole, Sulfanilamide, Pyrimethamine, Trimethoprim, Mupirocin and any combination thereof.

16. A method of obtaining the nonwoven fibre mat described in any of clauses 1 to 15, that comprises the steps of:

- coaxial electrospinning a:

   (a) solution of at least one biocompatible polymer to form the outer layer; and

   (b) a solution of molecules of at least one antineoplastic agent in a solution of at least one biocompatible polymer simultaneously, and

- desiccate the nonwoven fibre mat.

17. The method according to the preceding clause, wherein the biocompatible polymer concentration of step (a) is from 5 to 50 weight% of the solution.

18. The method according to any of the preceding clauses 16 to 17, wherein the biocompatible polymer of step (a) is solved in a solvent selected from: DMSO, DMF (N,N-dimethylformamide), hydrochloric acid (HCI), TFE (2,2,2,2-trifluoroethanol), HFIP (1,1,1,3,3,3,3-hexafluoro-2-propanol) and formic acid/acetic acid/chloroform.

19. The method according to any of the preceding clauses 16 to 18, wherein the biocompatible polymer is polycaprolactone.

20. The method according to any of the preceding clauses 16 to 19, wherein the biocompatible polymer is polycaprolactone and the antineoplastic agent is selected from 5-fluorouracil, methotrexate, irinotecan and any combination thereof.

21. The method according to any of the preceding clauses 16 to 20, wherein the step (c) comprises a desiccation at room temperature in a desiccator a period ranging between 10hr and 72hr and/or under vacuum.

22. The method according to any of the preceding clauses 16 to 21, wherein the power supply of the coaxial electrospinning is between 20,5 kV to 27 kV.

23. The method according to any of the preceding clauses 16 to 22, wherein electrospinning flow rates are selected from 0,03 mL/h and 0,30 mL/h, for the outer and inner layer.

24. The method according to any of the preceding clauses 16 to 23, wherein the electrospinning is carried out for a time between 0.5 h and 4 h.

25. The method according to any of the preceding clauses 16 to 24, wherein the electrospinning needles are from 15 G to 20 G to make the outer layer and from 22 G to 30 G to make the inner layer.

26. The nonwoven fibre mat according to any of the clauses 1-15 obtainable by the method described in any of the clauses 16 to 25.

27. The nonwoven fibre mat according to any of the clauses 1-15 or obtainable by the method described in any of the clauses 16 to 25 for use in medicine.

28. The nonwoven fibre mat according to the preceding clause, for use in the local delivery of an antineoplastic agent in a controlled and sustained manner to a body area to be treated.

29. The nonwoven fibre mat according to the preceding clause, wherein the body area is selected from skin, mucosas, bones, muscles, internal organs and solid tumors and any combination thereof.

30. The nonwoven fibre mat according to any of the preceding clauses 28 or 29, wherein the body area must have previously been subjected to surgery to resect a solid tumor.

31. The nonwoven fibre mat according to any of the preceding clauses 28 to 30, wherein the solid tumor is selected from: gastric adenocarcinoma, duodenal adenocarcinoma, mucinous cystoadenocarcinoma of the appendix, adenocarcinoma of the appendix, adenocarcinoma of the colon, adenocarcinoma of the rectum, epidermoid carcinoma of the rectum, adenocarcinoma of the anus, epidermoid carcinoma of the anus, colon adenocarcinoma, pancreatic cancer, hepatocarcinoma, hepatoblastoma, cholangiocarcinoma, hepatic cystoadenocarcinoma, hepatic epitheloid haemangioendothelioma, hepatic angiosarcoma, hepatic fibrosarcoma, hepatic leiomyosarcoma, hepatic metastases of colorectal carcinoma, hepatic metastases of endocrine carcinoma, hepatic metastases of non-colorectal and non-endocrine origin, pancreatic adenocarcinoma, serous cystoadenocarcinoma of pancreas, mucinous cystoadenocarcinoma of pancreas, papillary intraductal mucinous carcinoma of pancreas, acinar cell carcinoma of pancreas, pancreatoblastoma, pseudopapillary solid carcinoma, peritoneal carcinomatosis of ovarian origin, peritoneal carcinomatosis of gastric origin, peritoneal carcinomatosis of colorectal origin, pseudomyxoma peritoneum, mesothelioma, liposarcoma, malignant fibrohistiocytoma, leiomyosarcoma, paraganglioma, undifferentiated sarcoma, fibrosarcoma, retriperitoneal sarcoma, rhabdomyosarcoma, non-metastatic undifferentiated carcinoma, neuroblastoma, malignant schwanoma, benign schwanoma, ganglioneuroma, and/or lymphangioma.

The following examples illustrate the invention and should not be considered as limiting its scope of application

**Brief description of the figures**

**Figure 1** scanning electron microscopy (SEM) images of the different electrospun configurations methods: Upper line, three individual examples of the biocompatible nanofibers made with PCL without an antineoplastic drug (white bar represents 8 micrometers), Middle line, three individual examples of the biocompatible nanofibers made with PCL loaded with MTX (white bar represents 4 micrometers), Bottom line, three individual examples of the biocompatible nanofibers made with PCL loaded with 5FU (white bar represents 6 micrometers).

**Figure 2** scanning electron microscopy (SEM) images of biocompatible nanofibers made with PCL loaded with irinotecan. White bar represents 30 micrometers. Upper line: PIRI1, middle line PIRI2, bottom line PIRI3, each column is each of the three electrospinning parameters of table 2, left column: A, middle column, B and right column, C.

**Figure 3** FTIR spectrum of: a) coaxial nonwoven fibre mats without antineoplastic drug analysed right after being electrospun (Co 0h) or let at room conditions for 24 hours before performing the FTIR (Co 24h), b) coaxial nonwoven fibre mats without antineoplastic drug let at room conditions for 24 hours before performing the FTIR (Co 24h), acetic acid and formic acid; c) the PCL pellet and a coaxial polymeric mat desiccated for 24 hours (Co 24h Desiccated).

**Figure 4** Differential Scanning Calorimetry scans of: a) PCL pellets and a PCL scaffolds; b) drugless PCL mats, pure MTX and three MTX-loaded nonwoven fibre mat; c) drugless PCL mats, pure 5F and three 5F-blended nonwoven fibre mat. The nonwoven mats were obtained by electrospinning using different parameters.

**Figure 5** Differential Scanning Calorimetry drugless PCL mats, pure irinotecan and two nonwoven fibre mat formed by biocompatible nanofibers comprising irinotecan obtained by electrospinning using different parameters.

**Figure 6** Comparison of the FTIR spectrum of the a) PCL pellet and pure MTX, b) three MTX-loaded nonwoven fibre mat, c) PCL pellet and pure 5F and d) three 5F-loaded nonwoven fibre mat

**Figure 7** Comparison of the FTIR spectrum of the PCL pellet and the pure drug with the nonwoven fibre mats of the invention, being loaded with Irinotecan a) PIRI1, b) PIRI2.

**Figure 8** Drug release profile of the nonwoven fibre mats of the invention loaded with a) MTX and a) 5F.

**Figure 9** Drug release profile of the nonwoven fibre mats of the invention loaded with irinotecan: PIRI1, PIRI2 AND PIRI3. Black dot represents the amount of remaining irinotecan (% compared to the initial concentration) at day 7 and or 14.

**EXAMPLES**

**Materials and methods**

*Materials*

Polycaprolactone (PCL, Mw = 100 000 g/mol), 99% Chloroform, 100% Methanol, 98-100% Formic Acid and 99% Acetic Acid were purchased from Sigma Life Sciences, USA. 5-Fluouracil (5F) was purchased from AK Scientific Inc, USA, and Methotrexate (MTX) from a commercial provider.

*PCL nanofibers Coaxial Electrospinning*

Electrospinning solutions were prepared by dissolving 9 w.t. % to 16 w.t.% of PCL in formic acid/acetic acid/chloroform with a ratio of 47.5: 47.5: 5 (v/v/v), respectively. The chloroform in the solving solution improves the subsequent electersopun of the biopolymer containing solution. The biocompatible polymer solution was stirred at room temperature overnight and used within the first 72 hours. A second solution was prepared for each drug, 5F, MTX and irinotecan, by adding 28 mg/mL, 8.4mg/mL or 7mg/mL respectively to the original polymeric solution.

Once the solutions were ready, coaxial electrospinning was used to electrospun the nanofibers. The set-up consisted of a coaxial nozzle with an inner gauge of 26G and an outer gauge of 18G (Ramé-Hart Instrument Co, USA), two syringe pumps (KD Scientific Inc, USA) and a high voltage DC power supply (Heinzinger, Germany). Three different configurations were used to fabricate the fibres. The parameters used for each configuration tested are shown in Table 1. The scaffolds fabricated only with the biocompatible polymer solution were used as control and identified with the code CoPP. The drug-loaded fibres were fabricated using either of the antineoplastic drug-solutions in the inner tube and the polymeric solution in the outer one. The fibres loaded with 5F were identified as P5F and the ones loaded with MTX as PMTX.

Table 1 Electrospinning fabrication parameters of the three different configurations, with and without MTX and 5Fdrugs

| Sample code | Collector-nozzle distance (cm) | Power Supply (kV) | Outer flow (mL/h) | Inner flow (mL/h) | Time (h) |
|---|---|---|---|---|---|
| CoPP1/P5F1/PMTX1/ | 13.5 | 21 | 0.20 | 0.05 | 1.4 |
| CoPP2/P5F2/PMTX2 | 13.5 | 21 | 0.15 | 0.05 | 1.8 |
| CoPP3/P5F3/PMTX3 | 13.5 | 21 | 0.10 | 0.05 | 2.4 |

For Irinotecan, a different approach was used: In the first electrospinning fabrication round, for the PIRI1 meshes, in the external flow was used the biocompatible polymer solution (14w.t. % PCL) and the same solution with Irinotecan (7mg/mL) in the internal flow. In the second fabrication round, some configurations were changed. For some meshes (PIRI2) the same parameters as PIRI1 were used but with a 10 w.t. % PCL biocompatible polymer solution. Table 2. For others (PIRI3) the external flow contains also Irinotecan, that is, biocompatible polymer solution (10w.t. %) with drug in external and internal flows.

For every composition containing irinotecan (PIRI1, PIRI2 and 2PIRI3) three different electrospinning parameters were tested: A, B and C

Table 2 Electrospinning fabrication parameters of the three different configurations, with and without irinotecan

| Sample code | | Collector-nozzle distance (cm) | Power Supply (kV) | Outer flow (mL/h) | Inner flow (mL/h) | Time (h) |
|---|---|---|---|---|---|---|
| PIRI1 | A | 13.5 | 21 | 0.15 | 0.05 | 1.8 |
| PIRI2 | B | 13.5 | 21 | 0.20 | 0.05 | 1.4 |
| PIRI3 | C | 13.5 | 21 | 0.25 | 0.05 | 1.4 |

*Nanofibers Characterization: Surface morphology*

To assess the morphology of the biocompatible spun nanofibers, scanning electron microscopy (SEM, Phenom G2 Pro, USA) was used. The diameter of the biocompatible nanofibers was measured by using Image J software (National Institute of Health). A total of 4 images per scaffold were analyzed by measuring the diameter of 25 biocompatible nanofibers per frame. Therefore, the diameter of 100 nanofibers were measured randomly in each sample and the average diameter was reported. Nanofiber mats porosity and pore size was measured also by image analysis, using Diameter J, an Image J package. The obtained values are expressed as mean value $\pm$ standard deviation.

*Nanofibers Characterization: Solvent remanence*

Fourier Transform Infrared Spectroscopy (FTIR) was undertaken on pure PCL pellets and the three solvents (formic acid, acetic acid and chloroform), as well as on all the electrospun mat samples by the FTIR Spectrum 100 with the ATR (Perkin Elmer, USA). The scanning range tested goes from 650 to 4000 cm-1 with a total amount of 8 accumulations. In order to perform the FTIR analysis, solid samples were compressed whereas liquid samples were not. Mat samples were analyzed just after being fabricated or after 24 hours. In the case of the latter, the electrospun mats were either store overnight at room temperature or desiccated in a desiccator for the 24 hours.

*Drug loading efficiency: Differential Scanning Calorimetry (DSC)*

Differential Scanning Calorimetry was used to evaluate the drug integration inside the biocompatible nanofibers (DSC, 4000 Perkin Elmer). Samples with a mass between 5 and 20 mg were used for the analyses. PCL pellets were used without any further modification whereas scaffold pieces were cut and weighted in order to normalize the results. The analyzed temperature ranged from 20 to 100°C, with a temperature increase of 10°C/min, a one-minute break at 100 °C and the same conditions for the return to the initial temperature. The initial calorimeter flow used was 20 mWh$^{-1}$

*Drug loading efficiency: FTIR analysis*

Drug loading was semiquantitatively assessed by FTIR analysis of the drug-loaded scaffolds. A compressed sample of each drug-loaded mat was analyzed once the solvent was fully evaporated from the biocompatible nanofibers. The same equipment, range, and number of accumulations as in solvent perdurability characterization were used in these analyses.

*Drug loading efficiency: In vitro drug release*

The drug release of the different mat types was determined by immersing the loaded nanofibers in PBS. The procedure was as follows: three samples from each configuration for both 5F and MTX mats were cut into 2.25 cm$^2$ squares and submerged for 2 hours in MiliQ water, in order to remove the materials that could be outside the biocompatible nanofibers. The mats were then dried and weighted, before being introduced in 15 mL of PBS (pH = 7.4) for two weeks at 37°C on an orbital shaker (Polymax 1040 from Heidolph, Germany). During this period, spectrophotometry was used to characterize drug release: 100 $\mu$l of the immersing PBS were taken for absorbance reading every 24 hours. In order to determine the released drug amount, the respective calibration curve was obtained for each drug. Beer-Lambert equations were used to calculate drug concentration at $\lambda = 270$ nm and $\lambda = 350$ nm for 5F and MTX respectively. The fitted line for calibration of 5F in PBS is according to Equation 1 and the R$^2$ value is 0.9411. On the other hand, MTX calibration followed Equation 2, with a 0.9164 R$^2$ value.

$$Y = 3.5564x^{0.0086} \qquad \text{(Equation 1)}$$

$$Y = 6.9286x + 3.8287 \qquad \text{(Equation 2)}$$

For irinotecan, $\lambda$ = 300 nm. The fitted line for calibration of irinotecan in PBS is according to Equation 3 and the $R^2$ value is 0.9858.

$$Y = 2,3437x + 0,2639 \qquad \text{(Equation 3)}$$

*Statistical analysis*

To end up with the statistical analysis, independent student's t-test was performed to compare the effect of drugs and different electrospinning configurations on fibre diameter.

The statistical significance criterion was the same for all the analyses. Statistical difference was determined as not significant (p-value > 0.05), significant (0.01 < p-value < 0.05), very significant (0.001 < p-value < 0.01) or extremely significant (p-value < 0.001).

**Results**

***Surface Morphology***

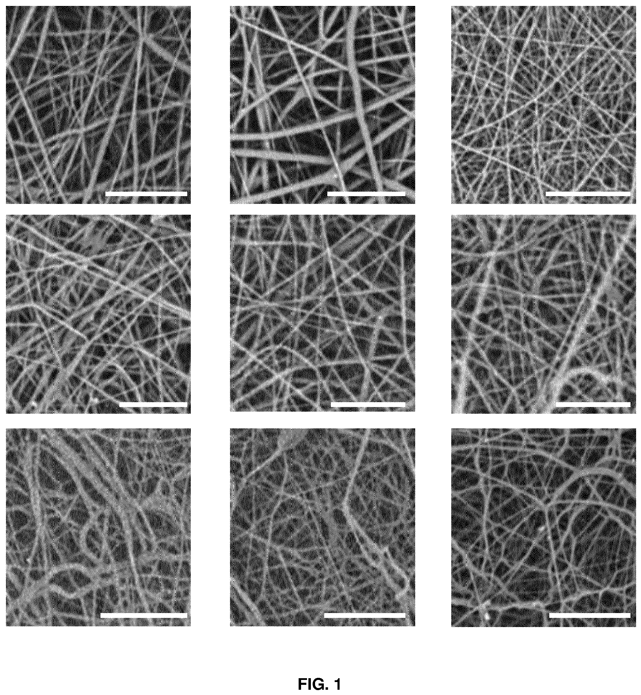

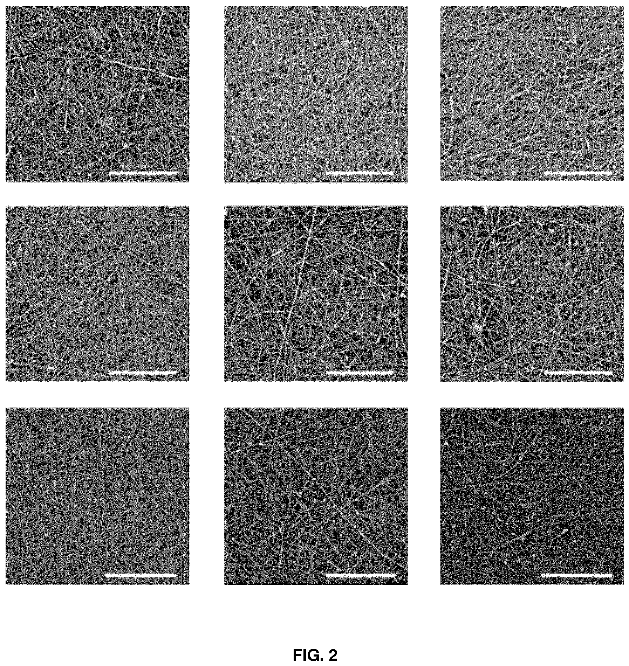

SEM was used to observe the nanofibers morphology and diameter for the different configurations tested with and without the drugs. The images obtained by SEM (Figure 1 and 2) showed that all configurations produced nanofibers, which had a great surface to volume ratio. No drug crystals or other deformations such as beads were observed in any of the images obtained, as all polymeric mats presented homogeneous fibres of different diameters. All fibres were in the nanofiber range as the maximum average diameters were less than 450 microns. The mats with greater diameters were the ones without drug encapsulation (Table 3), as all three samples had thicker fibres than any of the mats blended with drug. These differences were probably caused due to the conductivity differences between PCL and the drugs. In addition, the fibre distributions for the scaffolds were overall a Gaussian distribution positively skewed.

Table 3 Results of SEM images analysis on morphology (U = Uniform, B = beaded), fibre diameter and porosity, according to configuration and blended drug.

| Code | Morphology | | | Diameter | | | Porosity (%) | | |
|---|---|---|---|---|---|---|---|---|---|
| | Ctrl | 5F | MTX | Ctrl | 5F | MTX | Ctrl | 5F | MTX |
| 1 | U | U | u | 0.26±0.11 | 0.16±0.06 | 0.20±0.09 | 68.87 | 63.18 | 50.75 |
| 2 | U | U | U | 0.37±0.18 | 0.16±0.05 | 0.17±0.06 | 56.25 | 50.27 | 60.24 |
| 3 | U | U | U | 0.20±0.08 | 0.17±0.04 | 0.15±0.05 | 75.33 | 64.10 | 56.52 |

Significant differences were observed in the fibre diameter when outer flow was decreased from 0.20 mL/h to 0.15 mL/h in the case of control scaffolds, as CoPP1 mats vs CoPP2 ones showed a p-value = $2.40 \times 10^{-20}$. Also, reducing the outer flow from 0.15mL/h to 0.10 mL/h, significantly reduced the fibre diameter, between CoPP2 and CoPP3 (p-value = $8.07 \times 10^{-14}$). When the polymeric solutions were blended with 5F, no significant differences were observed if the outer flow was reduced from 0.20 mL/h to 0.15mL/h nor from 0.15 mL/h to 0.10 mL/h. On the other hand, introducing 5F into the mats significantly reduced the fibre diameter with respect to the control scaffolds: when the homologous samples were compared, extremely significant difference was observed in the three cases with p-values of $6.84 \times 10^{-13}$, $4.76 \times 10^{-20}$ and $4.17 \times 10^{-4}$ for CoPP1 vs P5F1, CoPP2 vs P5F2 and CoPP3 vs P5F3, respectively. Similar behaviours were observed in the case of the MTX scaffolds, as all the MTX-blended fibres had extremely significant smaller diameter than the pure polymeric ones (p-vales < 0.001). Moreover, as for the control polymeric scaffolds, a relationship between the total flow rate and the fibre diameter was observed in MTX-loaded scaffolds. In this case, reducing the outer flow from 0.20 mL/h to 0.10 mL/h significantly reduced the fibre diameter (p-value = $1.27 \times 10^{-6}$). This effect, which was not observed only in the case of 5F, could be due to solution conductivity. MTX is less polar than 5F. Therefore, it was more likely to behave as PCL, ending up in a fibre decreasing effect of the polymeric flow.

Porosity was also evaluated, obtaining porosities in the range of 45-75%. The drug loaded mats had overall less porous scaffolds, as two of the highest porosities obtained belonged to the control group (Table 3). This, together with the surface volume ratio improved the drug exposition and enhanced the interaction with the environment by an easy fluid flow through the scaffold.

In table 4 the results of the fibre mats loaded with irinotecan can be observed, similar results to the ones obtained with 5FU and MTX were obtained.

Table 4 Results of SEM images analysis on morphology (U = Uniform), fibre diameter and porosity, according to configuration and blended drug.

| Config. | Morphology | | | Diameter (μm) | | | Porosity (%) | | |
|---|---|---|---|---|---|---|---|---|---|
| | PIRI1 | PIRI2 | PIRI3 | PIRI1 | PIRI2 | PIRI3 | PIRI1 | PIRI2 | PIRI3 |
| A | U | U | U | 0.19±0.04 | 0.16±0.03 | 0.15±0.03 | 57.36 | 48.35 | 51.56 |
| B | U | U | U | 0.21±0.05 | 0.19±0.05 | 0.17±0.05 | 46.25 | 53.97 | 60.65 |
| C | U | U | U | 0.18±0.05 | 0.18±0.03 | 0.14±0.02 | 64.58 | 60.10 | 53.58 |

***Solvent remanence***

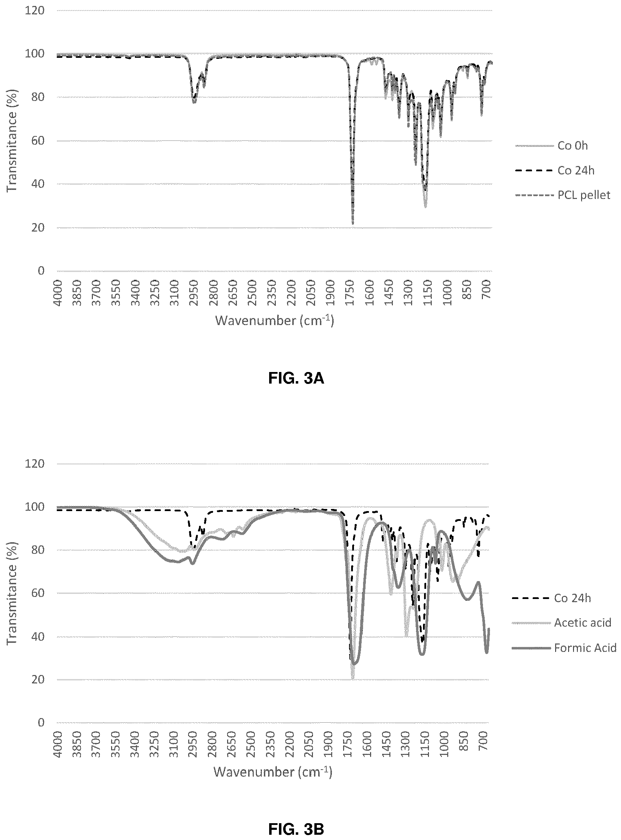

The non-desiccated mats did not show exactly the same spectrum as the pellet, as they showed two peaks at 1509 and 1545 cm$^{-1}$, which correspond to the carboxyl group of the acids present in the solvents (Figure 3a and b). This meant that the solvent was not completely removed during the fabrication. Therefore, introducing the scaffold into a body right after being electrospun could cause several problems, as the solvents were not fully evaporated.

Increasing the waiting time to 24 hours reduced notoriously the solvent presence, as the depth of the peak at 1150 cm$^{-1}$ was more pronounced in the mat analysed just after the electrospinning process than in the mat let 24 hours under room conditions before performing the FTIR. But the solvent-related peaks were still noticeable.

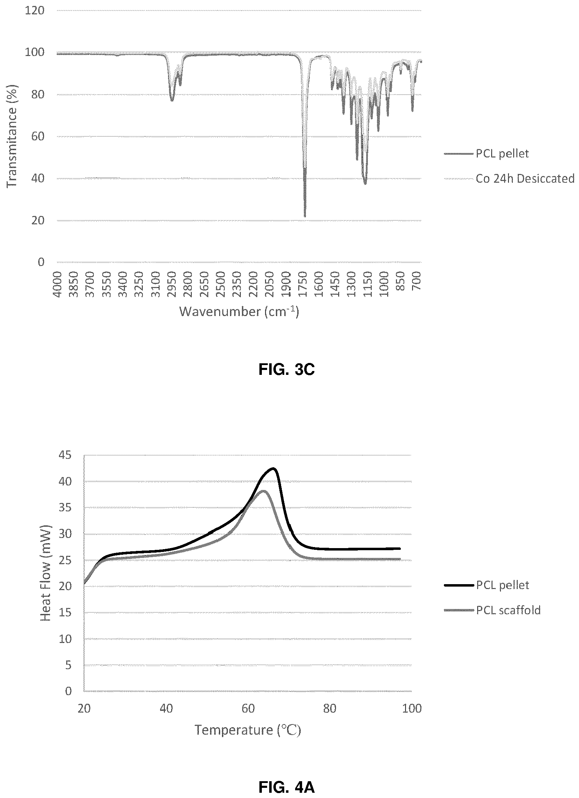

However, the solvent presence was completely erased after the mat was desiccated for 24 hours, as the curve overlapped exactly the same peaks of the curve of the pure polymer pellet (Figure 3c). This is possible because of the low-pressure environment generated in the desiccator. Therefore, it was proven that this widely used method was completely necessary to get rid of the solvents used in the electrospinning process.

***Antineoplastic Agent Loading***

The differential scanning calorimetry performed on PCL showed the melting transition of the polymer. No major differences were observed between a PCL mat against a pellet (Figure 4a), as both samples underwent a solid to liquid transition around 65°C. The only observable difference was the melting temperature and the heat flow, as the melting temperature was greater for the pellet than the mat: 66.1°C and 63.7°C, respectively. The heat flow for the pellet was overall higher. These values concur with the ones observed in the literature.

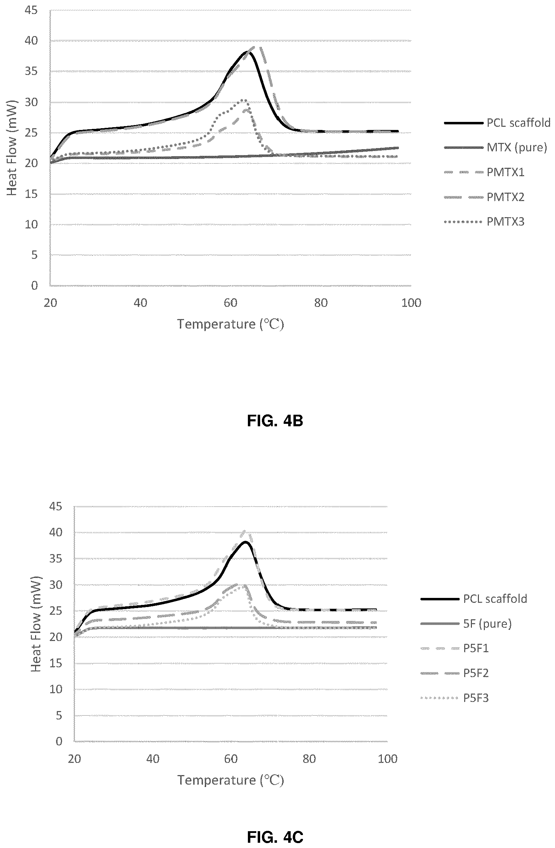

On the other hand, both pure MTX and 5F, did not show any transition in the analysed range (Figure 4 b and c), as the curves were completely flat. The scaffolds blended with these molecules showed a peak that was not present in the DSC performed on PCL, around 58°C. This peak was more notable in the mats blended with MTX. The observed melting point for the mats blended with MTX and 5F were between 62.9-66.0°C and 62.4-64.1, respectively. The observed difference between the control and the loaded scaffolds suggested that the drug loading occurred during the electrospinning process. Therefore, the DSC results suggested that both medicaments interacted with the polymer.

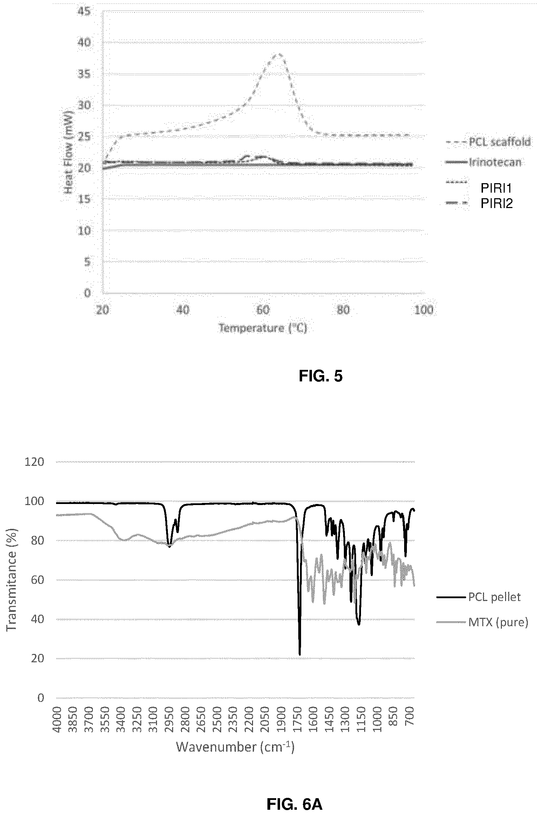

DSC results of nonwoven fibre mats containing irinotecan (Figure 5) show a peak at 59ºC, which represents the melting point of the polymer. At this moment, the polymer begins to crystallize and releases heat, so the heat flow immediately decays.

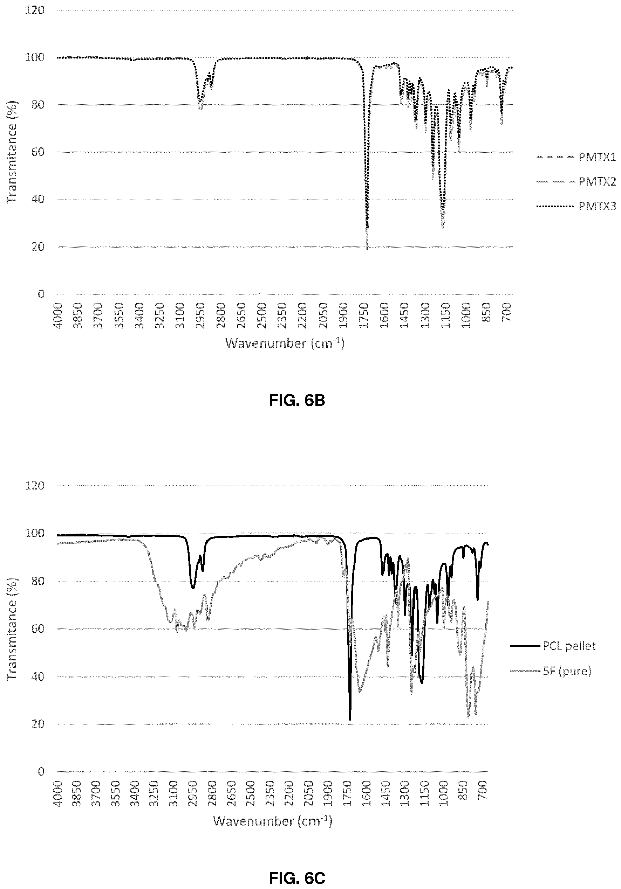

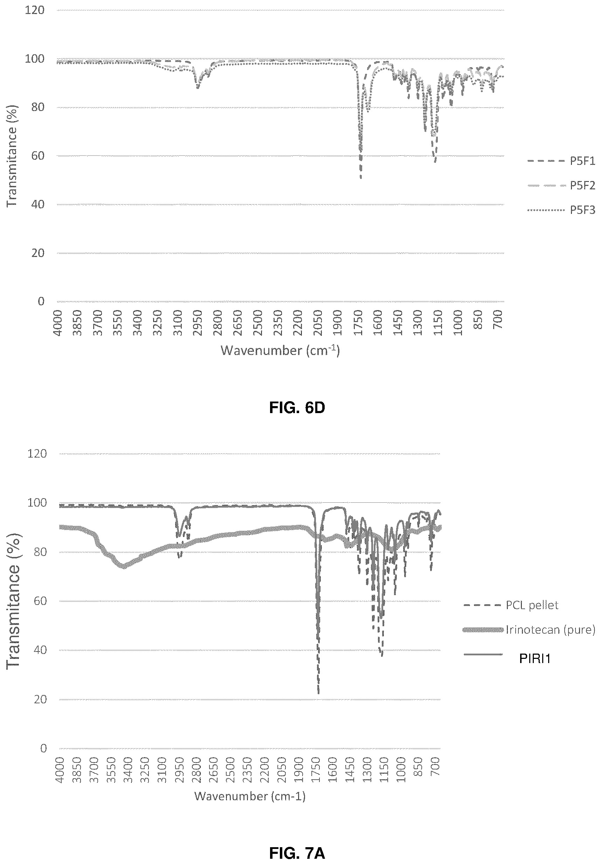

In addition to the DSC analysis, the infrared spectrum was used to observe de presence of MTX and 5F in the fibre mats. According to these FTIR results, it was concluded that a higher ratio of inner:outer flow in both MTX and 5F-loaded scaffolds was related to a greater drug concentration (Figure 6). As the outer solution did not contain the antineoplastic drug, a reduction of the outer flow related to the total solution flow, induced a greater drug flow, which meant a higher drug encapsulation. This trend was clearer in the fibres blended with 5F at 1661 cm$^{-1}$ and between 3000 and 3300 cm$^{-1}$, belonging that peak to the C=O double bond and the band to the N-H bond. In the case of MTX-loaded nonwoven fibre mats, the two ranges in which this drug presence was observable were from 1525 to 1617 cm$^{-1}$ and from 3000 to 3500 cm$^{-1}$, also due the C=O bond and the N-H bond.

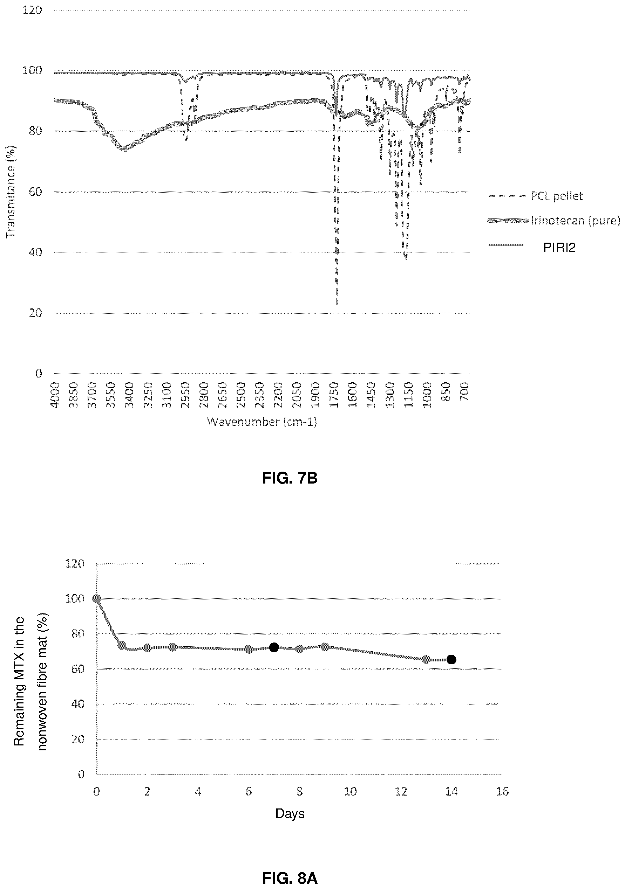

According to the Figure 7 a) and b), no residual solvent was found in the nonwoven fibres mats of the invention loaded with irinotecan (upper panel 1-IRI and bottom panel 2-IRI), since based on the literature the solvents used in this study visualize a peak around 700. This means that the manufacturing time and the volatility of the solvents are sufficient for their total evaporation. Even so, there are some differences between the depths of the peaks of the nonwoven fibre mat, which are probably effects of the configuration of each sample. The results of the other two types of mats were almost the same.

Taking into account that no drug crystals nor deformations on the fibres were observed in the SEM images, and the drug presence observed with both the DSC and FTIR analyses, it was depicted that the drug was successfully encapsulate during the coaxial electrospinning process performed in this invention.

*Antineoplastic Agent Release*

The release profile for MTX showed a high drug release in the first 24 hours, from there it showed a sustained drug concentration release from the first day until the end of the measurements, day 14 (Figure 8a). This fast and early release was due to drug diffusion rather than due to polymer degradation, because the first one is usually a dominant event in the early stages, while the latter is dominant in long-term release.

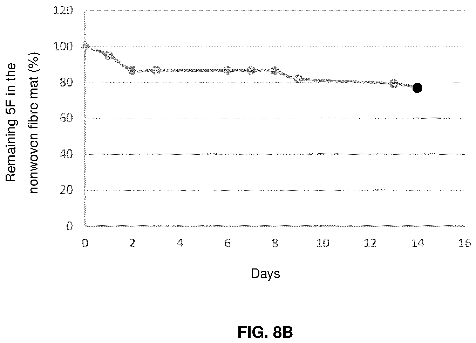

5F loaded nonwoven fibre mats have a different release profile (Figure 8b), They show a peak on day 2. from there it showed a sustained drug concentration release from the first day until the end of the measurements, day 14.

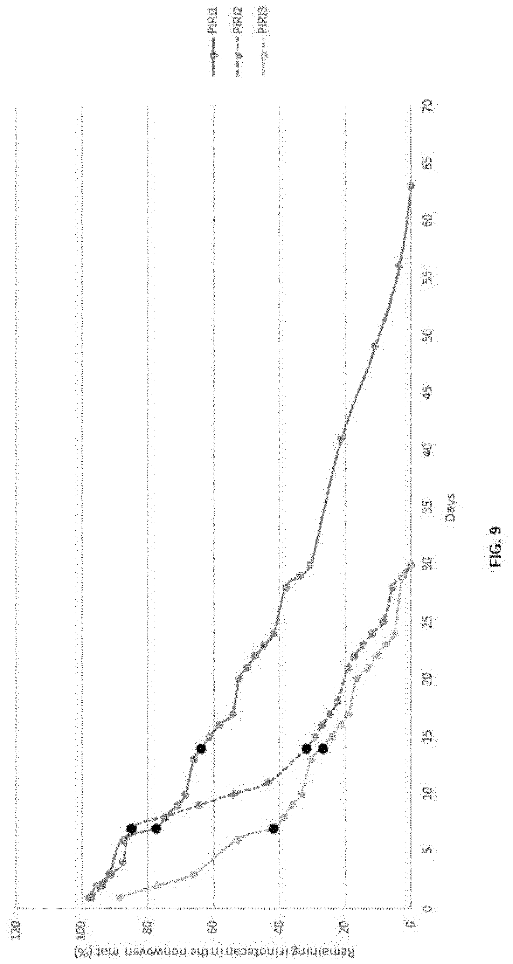

Analysing the release of the nonwoven fibre mat of the invention comprising irinotecan, wherein the concentration of PCL is 14w.t. % (PIRI1) it has been observed that the drug release speed is very slow during the first weeks (Figure 9) At day 7 it remains more than 80% of the drug, and at day 14 more than 63%. In total, with this nonwoven fibre mat more or less 0.5mg of Irinotecan were released for 56 days. It should be noted that another advantage of this system is that, as it is a local treatment, the necessary dose of drug for the patient is lower, as most of the drug is released directly into the affected area and its effectiveness is increased.

The curve of the PIRI2 meshes (PCL 10w.t. %) is similar to the previous one, but in this case the start of the release has been brought forward. It can be seen in the Figure 9. that in those samples it remains more than 84% of the drug until approximately first week the drug is little released, at day 7 from there the release speed increases, and at day 14 only 31% of the drug remains in the nonwoven fibre mat. In this case, the release of Irinotecan was of 0,45mg in 30 days.

Finally, PIRI3 nonwoven fibre mats, in addition to having PCL 10w.t. %, they have the drug in both the outer and inner layer, so the release is supposed to occur earlier than in the other two cases (Figure 9), At day 7 it remains less than 42% of the drug, and at day 14 less than 27% of the initial concentration of 0,54mg.

The measurement of both 5F, MTX and irinotecan release, demonstrated that the coaxial electrospinning process performed in the present invention generated nonwoven fibre mat that have a therapeutic use.

**Claims**

1. A nonwoven fibre mat formed by biocompatible nanofibers **characterized in that** the biocompatible nanofibers comprise:

   an outer layer composed by at least one biocompatible polymer, and
   an inner layer, composed by molecules of at least one antineoplastic agent and at least one biocompatible polymer, the inner layer being completely surrounded by the outer layer, so that the antineoplastic agent is encapsulated within the nanofibers,

   being both layers arranged concentrically being the diameter length of the outer layer between 35% and 65% of the total nanofiber diameter length, and the diameter length of the inner layer between 35% and 65% of the total nanofiber diameter length.

2. The nonwoven fibre mat according to the preceding claim, wherein the biocompatible nanofibers have at least one of the following parameter value:

   a diameter length between 100 and 300 microns,
   the melting point is between 45°C and 80°C and/or
   the porosity is between 50% to 75%

3. The nonwoven fibre mat according to any of the preceding claims, wherein the outer layer biocompatible polymer is selected from poly-glycolic acid, poly-D,L-lactic acid, poly-D,L-lactide-co-glicolide acid, polycaprolactone (PCL), polydioxanone, polyethylene glycol (PEG), polyurethanes (PU), polybenzimidazole (PBI), polycarbonate (PC), polyacrylonitrile (PAN), polyvinyl alcohol (PVA), polyethylene oxide (PEO), polystirene (PS), polyethylene terephtalate (PET), polyamide (PA), polyviniylphenol (PVP), polyvinylchloride (PVA, polyvinyl acetate and any combinations thereof and

the inner layer biocompatible polymer is selected from poly-glycolic acid, poly-D,L-lactic acid, poly-D,L-lactide-co-glicolide acid, polycaprolactone, polydioxanone polyethylene glicol (PEG), polyurethanes (PU), polybenzimidazole (PBI), polycarbonate (PC), polyacrylonitrile (PAN), polyvinil alcohol (PVA), polyethylene oxide (PEO), polystirene (PS), polyethylene terephtalate (PET), polyamide (PA), polyviniylphenol (PVP), polyvinylchloride (PVA, polyvinyl acetate and any combinations thereof.

4. The nonwoven fibre mat according to any of the preceding claims, wherein the antineoplastic agent is at least one anticancer drug selected from:

- DNA-damaging agents,
- mitosis inhibitors that do not directly affect DNA,
- external agents that affect cell cycle,
- monoclonal antibodies, and/or
- other anticancer agents.

5. The nonwoven fibre mat according to the proceeding claim, wherein the DNA-damaging agent is selected from at least one antimetabolite, and/or one camptothecin.

6. The nonwoven fibre mat according to any of the preceding claims, wherein the biocompatible polymer of the outer layer is polycaprolactone and the antineoplastic agent of the inner layer is selected from 5-fluorouracil, methotrexate and irinotecan and any combination thereof.

7. A method of obtaining the nonwoven fibre mat described in any of claims 1 to 6, that comprises the steps of:

- coaxial electrospinning a:

(a) solution of at least one biocompatible polymer to form the outer layer; and
(b) a solution of molecules of at least one antineoplastic agent in a solution of at least one biocompatible polymer simultaneously, and

- desiccate the nonwoven fibre mat.

8. The method according to the preceding claim, wherein the biocompatible polymer of step (a) is solved in a solvent selected from: DMSO, DMF (N,N-dimethylformamide), hydrochloric acid (HCl), TFE (2,2,2-trifluoroethanol), HFIP (1,1,1,3,3,3-hexafluoro-2-propanol) and formic acid/acetic acid/chloroform.

9. The method according to any of the preceding claims 7 to 8, wherein the biocompatible polymer is polycaprolactone and the antineoplastic agent is selected from 5-fluorouracil, methotrexate, irinotecan and any combination thereof.

10. The method according to any of the preceding claims 7 to 9, wherein the step (c) comprises a desiccation at room temperature in a desiccator a period ranging between 10hr and 72hr and/or under vacuum.

11. The method according to any of the preceding claims 7 to 10, wherein the electrospinning is carried out for a time between 0.5 h and 4 h, and/or
the electrospinning needles are from 15 G to 20 G to make the outer layer and from 22 G to 30 G to make the inner layer.

12. The nonwoven fibre mat according to any of the claims 1 to 6 obtainable by the method described in any of the claims 7 to 11.

13. The nonwoven fibre mat according to any of the claims 1 to 6 or obtainable by the method described in any of the claims 7 to 11 for use in medicine.

14. The nonwoven fibre mat according to the preceding claim, for use in the local delivery of an antineoplastic agent in a controlled and sustained manner to a body area to be treated, wherein the body area is selected from: skin, mucosas, bones, muscles, internal organs and solid tumors and any combination thereof.

15. The nonwoven fibre mat according to any of the preceding claims 27 or 28, wherein the body area must have previously been subjected to surgery to resect a solid tumor selected from: gastric adenocarcinoma, duodenal adenocarcinoma, mucinous cystoadenocarcinoma of the appendix, adenocarcinoma of the appendix, adenocarcinoma of the colon, adenocarcinoma of the rectum, epidermoid carcinoma of the rectum, adenocarcinoma of the anus, epidermoid carcinoma of the anus, colon adenocarcinoma, pancreatic cancer, hepatocarcinoma, hepatoblastoma, cholangiocarcinoma, hepatic cystoadenocarcinoma, hepatic epitheloid haemangioendothelioma, hepatic angiosarcoma, hepatic fibrosarcoma, hepatic leiomyosarcoma, hepatic metastases of colorectal carcinoma, hepatic metastases of endocrine carcinoma, hepatic metastases of non-colorectal and non-endocrine origin, pancreatic adenocarcinoma, serous cystoadenocarcinoma of pancreas, mucinous cystoadenocarcinoma of pancreas, papillary intraductal mucinous carcinoma of pancreas, acinar cell carcinoma of pancreas, pancreatoblastoma, pseudopapillary solid carcinoma, peritoneal carcinomatosis of ovarian origin, peritoneal carcinomatosis of gastric origin, peritoneal carcinomatosis of colorectal origin, pseudomyxoma peritoneum, mesothelioma, liposarcoma, malignant fibrohistiocytoma, leiomyosarcoma, paraganglioma, undifferentiated sarcoma, fibrosarcoma, retriperitoneal sarcoma, rhabdomyosarcoma, non-metastatic undifferentiated carcinoma, neuroblastoma, malignant schwanoma, benign schwanoma, ganglioneuroma, and/or lymphangioma.

FIG. 1

FIG. 2

FIG. 3A

FIG. 3B

**FIG. 3C**

**FIG. 4A**

FIG. 4B

FIG. 4C

**FIG. 5**

**FIG. 6A**

**FIG. 6B**

**FIG. 6C**

**FIG. 6D**

**FIG. 7A**

**FIG. 7B**

**FIG. 8A**

FIG. 8B

FIG. 9

Europäisches
Patentamt

European
Patent Office

Office européen
des brevets

## EUROPEAN SEARCH REPORT

Application Number

EP 21 38 3015

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | CN 110 327 283 A (UNIV NANJING FORESTRY) 15 October 2019 (2019-10-15) | 1,3-5,7, 8,10-15 | INV.<br>D04H1/728 |
| A | * the whole document * | 2,6,9 | D01D5/00<br>A61K9/00 |
| A | CN 111 991 343 A (UNIV SHANGHAI ENG SCIENCE) 27 November 2020 (2020-11-27) * the whole document * | 1-15 | D01F1/10 |

TECHNICAL FIELDS
SEARCHED     (IPC)

D04H
D01D
A61K
D01F
A61P

The present search report has been drawn up for all claims

| Place of search | Date of completion of the search | Examiner |
|---|---|---|
| The Hague | 8 June 2022 | Van Beurden-Hopkins |

CATEGORY OF CITED DOCUMENTS

X : particularly relevant if taken alone
Y : particularly relevant if combined with another
   document of the same category
A : technological background
O : non-written disclosure
P : intermediate document

T : theory or principle underlying the invention
E : earlier patent document, but published on, or
   after the filing date
D : document cited in the application
L : document cited for other reasons
  ...............................................................................
& : member of the same patent family, corresponding
   document

EPO FORM 1503 03.82 (P04C01)

## ANNEX TO THE EUROPEAN SEARCH REPORT
## ON EUROPEAN PATENT APPLICATION NO.

EP 21 38 3015

This annex lists the patent family members relating to the patent documents cited in the above-mentioned European search report.
The members are as contained in the European Patent Office EDP file on
The European Patent Office is in no way liable for these particulars which are merely given for the purpose of information.

08-06-2022

| Patent document cited in search report | | Publication date | Patent family member(s) | Publication date |
|---|---|---|---|---|
| CN 110327283 | A | 15-10-2019 | NONE | |
| CN 111991343 | A | 27-11-2020 | NONE | |

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- EP 2644191 A

### Non-patent literature cited in the description

- **CHAO LI ; QINGDE LI ; XIAOHUI NI ; GUOXIANG LIU ; WANLI CHENG ; GUANGPING HAN.** Coaxial Electrospinning and Characterization of Core-Shell Structured Cellulose Nanocrystal Reinforced PMMA/PAN Composite Fibers. *Materials (Basel),* June 2017, vol. 10 (6), 572
- **RUI CHEN ; CHEN HUANG ; QINFEI KE ; CHUANGLONG HE ; HONGSHENG WANG ; XIUMEI MO.** Preparation and characterization of coaxial electrospun thermoplastic polyurethane/collagen compound nanofibers for tissue engineering applications. *Colloids and Surfaces B: Biointerfaces,* 2010, vol. 79 (2), ISSN 0927-7765, 315-325
- **HE ZHAO ; YALI MA ; DAHUI SUN ; WENDI MA ; JIHANG YAO ; MEI ZHANG.** Preparation and Characterization of Coaxial Electrospinning rhBMP2-Loaded Nanofiber Membranes. *Journal of Nanomaterials,* 2019, vol. 2019, 13
- **AZIZ BABAPOOR ; GHOLAMREZA KARIMI ; SEYYED IMAN GOLESTANEH ; MEHDI AHMADI MEZJIN.** Coaxial electro-spun PEG/PA6 composite fibers: Fabrication and characterization. *Applied Thermal Engineering,* 2017, vol. 118, ISSN 1359-4311, 398-407